# EUROPEAN PATENT APPLICATION

(11) **EP 3 939 663 A1**
(43) Date of publication of application: **19.01.2022**
(21) Application number: 20771009.6
(22) Date of filing: 11.03.2020
(51) Int. Cl.: A61P 27/02, C12N 5/071, C12N 1/00, A61K 31/175

(54) **THERAPEUTIC AGENT FOR CORNEAL DISEASES**

(30) Priority: 12.03.2019 JP 2019044854
(71) Applicant: Nissan Chemical Corporation, Tokyo 103-6119 (JP)
(72) Inventor: FUKASAWA, Natsuki, Shiraoka-shi, Saitama 349-0294 (JP); NISHINO, Taito, Shiraoka-shi, Saitama 349-0294 (JP); WATANABE, Rina, Shiraoka-shi, Saitama 349-0294 (JP)
(74) Representative: J A Kemp LLP
(86) International application number: PCT/JP2020/010465
(87) International publication number: WO 2020/184605

(57) **Abstract**

The present invention relates to a novel means for treating corneal diseases. The present invention provides a therapeutic agent for corneal diseases, containing a compound represented by the following formula (I): 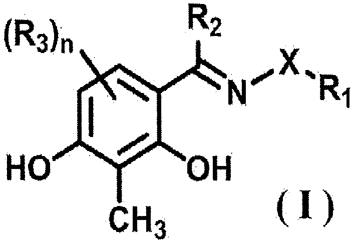 wherein each symbol is as defined in the DESCRIPTION, or a salt thereof.

## Description

### [Technical Field]

The present invention relates to a novel therapeutic agent for corneal diseases and a treatment method for corneal diseases using the same.

### [Background Art]

The cornea is one of the transparent layered tissues that constitute the eyeball. In the case of human, the cornea has a diameter of about 12 mm, a thickness of the central part of about 0.5 mm, and a thickness of the peripheral part of about 0.7 mm, and is composed of five layers of corneal epithelium, Bowman's membrane, stroma of the cornea, Descemet's membrane, and corneal endothelium from the surface. Corneal epithelial cells play a barrier function to prevent the invasion of exogenous substances, pathogenic bacteria, and the like, and when corneal epithelial cells are deficient due to various diseases or injury, not only pain and invisible conditions continue, but also complications such as infectious diseases and the like occur. On the other hand, corneal endothelial cells are indispensable for keeping the water content and thickness of the cornea constant and maintaining the transparency of the cornea. Lack of corneal endothelial cells leads to corneal opacity and causes a significant decrease in the visual acuity. Since corneal epithelial cells and corneal endothelial cells play an important role in eye function, the development of a therapeutic agent for diseases and injury that cause lack of these cells is desired.

As a treatment method for severe corneal diseases and injury, corneal transplantation therapy has been conventionally performed. However, for reasons that there is a shortage of donors who can provide the cornea needed for transplantation, a rejection reaction to the donor-derived cornea may occur, and the like, the development of a treatment method in which corneal epithelial cells and corneal endothelial cells are amplified in vitro and then transplanted has been desired (patent documents 1, 2).

In recent years, a low-molecular-weight compound that promotes the proliferation of various cells has been reported (patent document 3). However, the proliferation promoting effect of the compound on corneal epithelial cells and corneal endothelial cells has not been reported.

### [Document List]

### [Non-patent documents]

Patent document 1: WO 2013/012087
Patent document 2: WO 2013/100208
Patent document 3: WO 2019/022222

### [Summary of Invention]

### [Technical Problem]

The present invention aims to provide a medicament for treating corneal diseases, and a method for treating corneal diseases using the same.

### [Solution to Problem]

The present inventors have conducted intensive studies in an attempt to solve the above-mentioned problems and found that a specific compound promotes proliferation of corneal epithelial cells and corneal endothelial cells, and conducted further researches based on such finding, which resulted in the completion of the present invention.

That is, the present invention provides the following.
[1] A therapeutic agent for a corneal disease, comprising a compound represented by the following formula (I) or a salt thereof: 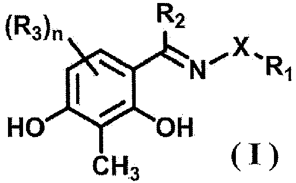 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.
[2] The therapeutic agent of [1], wherein
   R₂ is an alkyl group having 1 - 4 carbon atoms,
   n is 0,
   Ar is a phenyl group or naphthyl group, each optionally having a hydroxy group or a C₁₋₄ alkyl group,
   Y is a single bond, and
   Z is an alkylene group having 1 - 4 carbon atoms.
[3] The therapeutic agent of [1], wherein
   R₂ is an alkyl group having 1 - 4 carbon atoms,
   n is 0,
   Ar is a phenyl group or naphthyl group, each optionally having a halogen atom, a C₁₋₄ alkyl group, a hydroxy group, or a C₁₋₄ alkoxy group,
   Y is an alkylene group having 1 - 4 carbon atoms and substituted by an alkyl group having 1 - 4 carbon atoms, and
   Z is a single bond.
[4] The therapeutic agent of [1], wherein
   R₂ is a methyl group, an ethyl group, or an isobutyl group,
   n is 0,
   Ar is a phenyl group optionally having a hydroxy group or a methyl group, and
   Y is a methylene group optionally having a methyl group or an ethyl group.
[5] The therapeutic agent of [1], wherein
   R₂ is a methyl group, an ethyl group, or an isobutyl group,
   n is 0,
   Ar is a phenyl group optionally having a hydroxy group or a halogen atom, and
   Y is a methylene group optionally having a methyl group or an ethyl group.
[6] The therapeutic agent of [1], wherein
   R₂ is a methyl group, an ethyl group, or an isobutyl group,
   n is 0,
   Ar is a phenyl group optionally having a hydroxy group or a methyl group, and
   Y is a single bond.
[7] The therapeutic agent of [4], wherein Z is a single bond.
[8] The therapeutic agent of [5] or [6], wherein Z is a methylene group optionally having a methyl group or an ethyl group.
[9] The therapeutic agent of any of [1] to [8], wherein the corneal disease is selected from the group consisting of corneal epithelium disorder, superficial punctate keratopathy, cornea erosion, corneal ulcer, dry eye, corneal epithelial abrasion, aniridia, pterygium, sclerocornea, cornea dystrophy, corneitis, limbal stem cell deficiency, chemical burn, burn, Stevens-Johnson syndrome, ocular pemphigoid, diabetes keratopathy, bullous keratopathy, corneal endothelitis, Fuchs' corneal endothelial dystrophy, cornea guttata, iridocorneal endothelial syndrome, and post-corneal transplantation graft failure.
[10] A method for treating a corneal disease, comprising administering a compound represented by the following formula (I) or a salt thereof to a subject in need thereof: 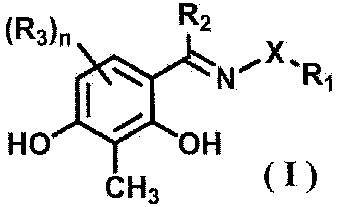 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.
[11] A method for promoting the proliferation of a corneal epithelial cell or a corneal endothelial cell, comprising culturing the corneal epithelial cell or the corneal endothelial cell in a medium composition comprising a compound represented by the following formula (I) or a salt thereof: 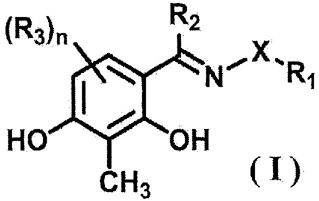 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.
[12] A method for producing corneal epithelial cells or corneal endothelial cells, comprising using the method of the aforementioned [11].
[13] A compound represented by the following formula: 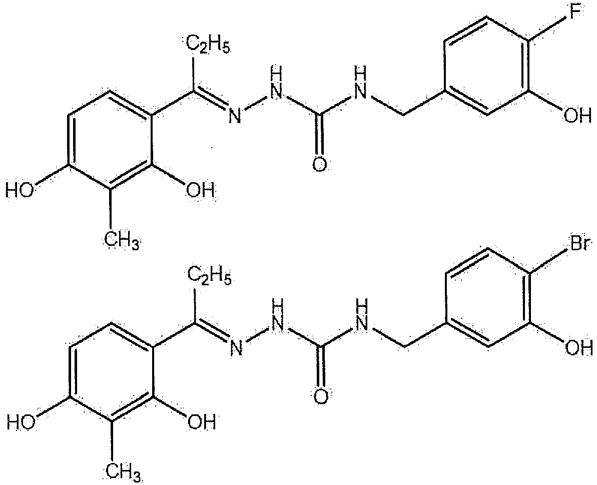 or
   or a salt thereof.
[14] A proliferation promoter of a corneal epithelial cell or a corneal endothelial cell, comprising a compound represented by the following formula (I) or a salt thereof: 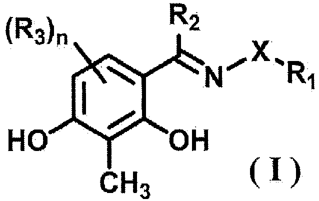 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.
[15] The proliferation promoter of [14], wherein
   R₂ is an alkyl group having 1 - 4 carbon atoms,
   n is 0,
   Ar is a phenyl group or naphthyl group, each optionally having a hydroxy group or a C₁₋₄ alkyl group,
   Y is a single bond, and
   Z is an alkylene group having 1 - 4 carbon atoms.
[16] The proliferation promoter of [14], wherein
   R₂ is an alkyl group having 1 - 4 carbon atoms,
   n is 0,
   Ar is a phenyl group or naphthyl group, each optionally having a halogen atom, a C₁₋₄ alkyl group, a hydroxy group, or a C₁₋₄ alkoxy group,
   Y is an alkylene group having 1 - 4 carbon atoms and substituted by an alkyl group having 1 - 4 carbon atoms, and
   Z is a single bond.
[17] The proliferation promoter of [14], wherein
   R₂ is a methyl group, an ethyl group, or an isobutyl group,
   n is 0,
   Ar is a phenyl group optionally having a hydroxy group or a methyl group, and
   Y is a methylene group optionally having a methyl group or an ethyl group.
[18] The proliferation promoter of [14], wherein
   R₂ is a methyl group, an ethyl group, or an isobutyl group,
   n is 0,
   Ar is a phenyl group optionally having a hydroxy group or a halogen atom, and
   Y is a methylene group optionally having a methyl group or an ethyl group.
[19] The proliferation promoter of [14], wherein
   R₂ is a methyl group, an ethyl group, or an isobutyl group,
   n is 0,
   Ar is a phenyl group optionally having a hydroxy group or a methyl group, and
   Y is a single bond.
[20] The proliferation promoter of [14], wherein Z is a single bond.
[21] The proliferation promoter of [18] or [19], wherein Z is a methylene group optionally having a methyl group or an ethyl group.

### [Effect of Invention]

According to the present invention, proliferation of corneal epithelial cells and corneal endothelial cells can be efficiently promoted. Therefore, according to the present invention, a disease or injury lacking corneal epithelial cells and corneal endothelial cells can be treated. Furthermore, corneal epithelial cells and corneal endothelial cells for transplantation can be efficiently produced in vitro.

### [Description of Embodiments]

The terms used in the present specification are defined in the following.

In the present specification, n- means normal, i- means iso, sec- means secondary and tert- means tertiary, respectively. In the present specification, o- means ortho, m-means meta, and p- means para, respectively.

The "halogen atom" is a fluorine atom, a chlorine atom, a bromine atom or an iodine atom. The "halogeno group" is fluoro, chloro, bromo, or iodo.

The "alkyl group having 1 - 6 carbon atoms" means a straight chain or branched alkyl group having 1 - 6 carbon atoms, and C₁₋₆ alkyl groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be specifically mentioned. As such alkyl group, a lower alkyl group having 1 - 4 carbon atoms (C₁₋₄ alkyl group) is preferred, and a methyl group and an ethyl group are particularly preferred.

Unless particularly limited, the "cyclic alkyl", preferably means a cycloalkyl group having 3 - 8 carbon atoms (C₃₋₈ cycloalkyl group) and, for example, cyclopropyl, cyclobutyl, cyclopentyl, cyclohexyl, cycloheptyl, cyclooctyl and the like can be mentioned. When "optionally containing a hetero atom in the ring" is indicated, a 3- to 8-membered saturated heterocyclyl group optionally containing one or more hetero atoms as a ring-constituting atom is also included. Examples of the 3- to 8-membered saturated heterocyclyl group include tetrahydrofuranyl, tetrahydropyranyl, aziridinyl, azetidinyl, pyrrolidinyl, piperidinyl, morpholinyl and the like.

The "aryl group" includes monocyclic, bicyclic, tricyclic and tetracyclic carbocyclic groups in which at least one ring is an aromatic group, and each ring has 5 - 8 ring atoms. Specifically, phenyl, indenyl, naphthyl, fluorenyl and the like can be mentioned. As the aryl group, C₆₋₁₀ aryl groups such as phenyl, indenyl, naphthyl and the like are preferred, phenyl and naphthyl are more preferred, and phenyl is particularly preferred.

The "alkylene group having 1 - 6 carbon atoms" means a straight carbon chain having 1 - 6 carbon atoms, and groups such as methylene, ethylene, propylene, butylene, pentylene, hexylene and the like can be specifically mentioned. As the alkylene group, an alkylene group having 1 - 4 carbon atoms is preferred, and methylene is more preferred.

The "heteroaryl group" includes monocyclic, bicyclic, tricyclic and polycyclic aromatic heterocyclic groups in which at least one ring atom is a hetero atom, and the remaining ring atoms are carbons. As the monocyclic heteroaryl group, a 5-membered or 6-membered aromatic heterocyclic group, in which at least one ring atom is a hetero atom and the remaining ring atoms are carbons, can be mentioned, though not limited thereto. Specific examples of such heteroaryl group include monocyclic aromatic heterocyclic groups such as furyl, thiophenyl, pyrrolyl, imidazolyl, pyrazolyl, oxazolyl, isoxazolyl, thiazolyl, isothiazolyl, triazolyl, tetrazolyl, thiadiazolyl, oxadiazolyl, pyridyl, pyridazinyl, pyrimidinyl, pyrazinyl and the like; and bicyclic or polycyclic aromatic heterocyclic groups such as indolyl, isoindolyl, indazolyl, benzofuryl, benzothiophenyl, benzimidazolyl, benzoxazolyl, benzothiazolyl, benzisothiazolyl, quinolyl, isoquinolyl, cinnolinyl, phthalazinyl, quinazolinyl, quinoxalinyl, pteridinyl, imidazooxazolyl, imidazothiazolyl, imidazoimidazolyl, benzooxadiazolyl, benzothiadiazolyl, benzoxazinyl, benzisoxazolyl, benzothiazinyl, furo[2,3-b]pyridyl, thieno[2,3-b]pyridyl, naphthyridinyl, imidazopyridyl, oxazolopyridyl, thiazolopyridyl, purinyl, carbazolyl, dibenzothiophenyl and the like.

The "optionally having substituent(s)" means being unsubstituted or having one or more substituents at substitutable positions. When two or more substituents are present, the respective substituents may be the same or different.

The "alkyl group having 1 - 6 carbon atoms", "aryl group" and "alkylene group having 1 - 6 carbon atoms" may each have a substituent, and as such substituent, the following groups can be mentioned. As the substituent of the "alkyl group having 1 - 6 carbon atoms", the following (1) - (40) can be mentioned, and as the substituent of the "aryl group" and "alkylene group having 1 - 6 carbon atoms", the following (1) - (41) can be mentioned.
(1) halogeno group,
(2) hydroxy group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, and each isomer thereof),
(7) alkynyl group (C₂₋₁₀ alkynyl group; e.g., ethynyl, propynyl, butynyl, pentynyl, hexynyl, and each isomer thereof),
(8) halogeno alkyl group (e.g., monofluoromethyl, difluoromethyl, trifluoromethyl, monofluoroethyl, difluoroethyl, trifluoroethyl, chloromethyl, chloroethyl, dichloroethyl, and each isomer thereof),
(9) cyclic alkyl group (optionally containing a hetero atom in the ring) (C₃₋₈ cycloalkyl group; e.g., cyclopropyl, cyclobutyl, cyclopentyl, cyclohexyl, etc., 3- to 8-membered saturated heterocyclyl groups; e.g., tetrahydrofuranyl, tetrahydropyranyl, aziridinyl, azetidinyl, pyrrolidinyl, piperidinyl, morpholinyl, etc.),
(10) aryl group (C₆₋₁₀ aryl group; e.g., phenyl, naphthyl),
(11) heteroaryl group (5- or 6-membered monocyclic heteroaryl group; e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), etc., bicyclic heteroaryl groups; e.g., benzofuryl, benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolyl, isoquinolyl, cinnolinyl, phthalazinyl, quinazolinyl, quinoxalinyl, pteridinyl, imidazooxazolyl, imidazothiazolyl, imidazoimidazolyl, etc.),
(12) alkoxy group (C₁₋₆ alkoxy group; e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (C₁₋₆ alkylthio group; e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (same as in the above-mentioned (12)) substituted by aryl group (same as in the above-mentioned (10)),
(15) alkylthio group (same as in the above-mentioned (13)) substituted by aryl group (same as in the above-mentioned (10)),
(16) alkoxy group (same as in the above-mentioned (12)) substituted by heteroaryl group (same as in the above-mentioned (11)),
(17) alkylthio group (same as in the above-mentioned (13)) substituted by heteroaryl group (same as in the above-mentioned (11)),
(18) cyclic alkyl (optionally containing a hetero atom in the ring) oxy group (C₃₋₈ cycloalkyloxy group; e.g., cyclopropyloxy, cyclobutyloxy, cyclopentyloxy, cyclohexyloxy, etc., 3- to 8-membered saturated heterocyclyl groups; e.g., tetrahydrofuranyloxy, tetrahydropyranyloxy, aziridinyloxy, azetidinyloxy, pyrrolidinyloxy, piperidinyloxy, morpholinyloxy, etc.),
(19) aryloxy group (e.g., group in which aryl group (same as in the above-mentioned (10)) is bonded to oxygen atom),
(20) heteroaryloxy group (e.g., group in which heteroaryl group (same as in the above-mentioned (11)) is bonded to oxygen atom),
(21) halogeno alkoxy group (e.g., group in which halogeno alkyl group (same as in the above-mentioned (8)) is bonded to oxygen atom),
(22) halogeno alkylthio group (e.g., group in which halogeno alkyl group (same as in the above-mentioned (8)) is bonded to sulfur atom),
(23) alkoxy group (same as in the above-mentioned (12)) substituted by hydroxy group,
(24) alkoxy group (same as in the above-mentioned (12)) substituted by alkoxy group (same as in the above-mentioned (12)),
(25) amino group,
(26) amino group mono- or di-substituted by alkyl group,
   Here, as the "alkyl group", C₁₋₆ alkyl group can be mentioned, and specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.
(27) carbamoyl group,
(28) carbamoyl group (e.g., methylcarbamoyl, ethylcarbamoyl, dimethylcarbamoyl, diethylcarbamoyl, ethylmethylcarbamoyl, etc.) mono- or di-substituted by alkyl group (same as "alkyl group" in the above-mentioned (26)),
(29) sulfamoyl group,
(30) sulfamoyl group (e.g., methylsulfamoyl, ethylsulfamoyl, dimethylsulfamoyl, diethylsulfamoyl, ethylmethylsulfamoyl, etc.) mono- or di-substituted by alkyl group (same as "alkyl group" in the above-mentioned (26)),
(31) alkanoyl group (e.g., group in which hydrogen atom or alkyl group (same as "alkyl group" in the above-mentioned (26)) is bonded to carbonyl group),
(32) aroyl group (e.g., group in which aryl group (same as in the above-mentioned (10)) is bonded to carbonyl group),
(33) alkylsulfonylamino group (e.g., sulfonylamino group substituted by alkyl group (same as "alkyl group" in the above-mentioned (26))),
(34) arylsulfonylamino group (e.g., sulfonylamino group substituted by aryl group (same as in the above-mentioned (10))),
(35) heteroarylsulfonylamino group (e.g., sulfonylamino group substituted by heteroaryl group (same as in the above-mentioned (11))),
(36) acylamino group (e.g., amino group substituted by acyl group),
   Here, the "acyl group" is, for example, a group in which hydrogen atom or C₁₋₆ alkyl group is bonded to carbonyl group (C₁₋₇ alkanoyl group), a group in which C₆₋₁₀ aryl group is bonded to carbonyl group (C₇₋₁₁ aroyl group), or the like. Here, the "C₁₋₆ alkyl group" is the above-mentioned "alkyl group" having 1 - 6 carbon atoms, and the "C₆₋₁₀ aryl group" is the above-mentioned "aryl group" having 6 - 10 carbon atoms. As the acyl group, formyl group, acetyl group, propionyl group, butyryl group, isobutyryl group, valeryl group, isovaleryl group, pivaloyl group, hexanoyl group, acryloyl group, methacryloyl group, crotonoyl group, isocrotonoyl group, benzoyl group, naphthoyl group and the like can be specifically mentioned,
(37) alkoxycarbonylamino group (e.g., carbonylamino group substituted by alkoxy group (same as in the above-mentioned (12))),
(38) alkylsulfonyl group (e.g., sulfonyl group substituted by alkyl group (same as "alkyl group" in the above-mentioned (26))),
(39) alkylsulfinyl group (e.g., sulfinyl group substituted by alkyl group (same as "alkyl group" in the above-mentioned (26))),
(40) alkoxycarbonyl group (e.g., methoxycarbonyl group, ethoxycarbonyl group),
(41) alkyl group (C₁₋₆ alkyl group; e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl, etc.), and the like.

When two or more substituents are present, they may be the same or different.

Specific compounds used in the present invention are shown by the following formula (I). A compound represented by 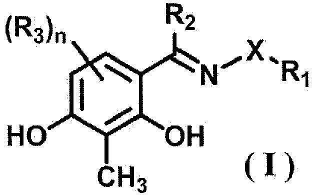 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}, or a salt thereof (hereinafter a compound represented by the formula (I) and a salt thereof are sometimes generically referred simply to as "the specific compound used in the present invention", "the compound forming the present invention", and the like).

The compound represented by the formula (I) may be in a salt form. Examples of the salt of the compound represented by the aforementioned formula (I) include salts with inorganic acids such as hydrochloric acid, hydrobromic acid and the like, and salts with organic acids such as acetic acid, propionic acid, tartaric acid, fumaric acid, maleic acid, malic acid, oxalic acid, succinic acid, citric acid, benzoic acid and the like, and the like. These salts are produced by known methods.

The compound represented by the formula (I) may have, depending on the kind of the substituent, geometric isomers of an E-form having an E configuration and a Z-form having a Z configuration. The present invention encompasses these E-form, Z-form, and a mixture containing E-form and Z-form at any proportion.

In addition, the compound represented by the formula (I) may have an optically active form due to the presence of one or more asymmetric carbon atoms. The compound represented by the formula (I) encompasses all optically active forms and racemates.

### [1. Therapeutic agent for corneal diseases]

The present invention provides therapeutic agents for corneal diseases and injury, containing a compound represented by the following formula (I) or a salt thereof (hereinafter to be sometimes referred to as "therapeutic agent for corneal diseases" or "the therapeutic agent of the present invention", etc.). 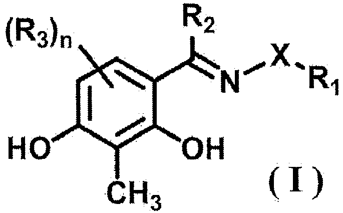 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.

In one embodiment, when Y is a single bond, Z is an alkylene group optionally having substituent(s) (more preferably, an alkylene group without a substituent, particularly preferably, a methylene group), Ar is an aryl group optionally having a substituent (more preferably, a halogen atom, a methyl group, a hydroxy group, or a methoxy group) (more preferably, an aryl group having a hydroxy group or an aryl group without a substituent, particularly preferably, a phenyl group or a phenyl group having a hydroxy group), R₂ is an alkyl group having 1 - 6 carbon atoms and optionally having substituent(s) (more preferably, an alkyl group having 1 - 6 carbon atoms and without a substituent, particularly preferably, an ethyl group), and n is 0, 1, or 2, preferably, 0.

In addition, when Y is an alkylene group having 1 - 6 carbon atoms and optionally having substituent(s) (more preferably, an alkylene group having 1 - 6 carbon atoms and having a substituent, particularly preferably, a methylene group substituted by a methyl group or an ethyl group), Z is a single bond, Ar is an aryl group optionally having substituent(s) (more preferably, aryl group having a substituent, particularly preferably, a phenyl group or naphthyl group, each having a halogeno group, a methyl group, a hydroxy group, or an ethoxy group), R₂ is an alkyl group having 1 - 6 carbon atoms and optionally having substituent(s) (more preferably, alkyl group having 1 - 6 carbon atoms and without a substituent, particularly preferably, a methyl group, an ethyl group, or an isopropyl group), and n is 0, 1, or 2, preferably, 0.

Examples of a specific preferred embodiment of a compound represented by the formula (I) include the following compounds.

### [Compound (I-1)]

A compound represented by the formula (I) wherein, in the aforementioned formula (I),
X is -NHCO-,
R₁ is -Y-NH-Z-Ar, wherein Y is a single bond, Z is an alkylene group having 1 - 6 carbon atoms (C₁₋₆ alkylene group), Ar is a C₆₋₁₀ aryl group optionally having a halogen atom, a C₁₋₆ alkyl group, a hydroxy group, or a C₁₋₆ alkoxy group,
R₂ is an alkyl group having 1 - 6 carbon atoms (C₁₋₆ alkyl group),
R₃ is a hydroxy group, and n is 0, 1, or 2.

### [Compound (1-2)]

A compound represented by the formula (I) wherein, in the aforementioned formula (I),
X is -NHCO-,
R₁ is -Y-NH-Z-Ar, wherein Y is a single bond, Z is an alkylene group having 1 - 4 carbon atoms (C₁₋₄ alkylene group), Ar is a phenyl group or naphthyl group, each optionally having a hydroxy group or a C₁₋₄ alkyl group,
R₂ is an alkyl group having 1 - 4 carbon atoms (C₁₋₄ alkyl group; e.g., methyl, ethyl, n-propyl, isopropyl, n-butyl, isobutyl, sec-butyl, tert-butyl), and
n is 0.

### [Compound (I-3)]

A compound represented by the formula (I) wherein, in the aforementioned formula (I),
X is -NHCO-,
R₁ is -Y-NH-Z-Ar, wherein Y is a single bond, Z is methylene, and Ar is a phenyl group optionally having a hydroxy group or a methyl group,
R₂ is methyl, ethyl, or isobutyl (preferably, ethyl), and
n is 0.

### [Compound (1-4)]

A compound represented by the formula (I) wherein, in the aforementioned formula (I),
X is -NHCO-,
R₁ is -Y-NH-Z-Ar, wherein Y is an alkylene group having 1 - 6 carbon atoms (C₁₋₆ alkylene group) and substituted by an alkyl group having 1 - 6 carbon atoms (C₁₋₆ alkyl group), Z is a single bond, Ar is a C₆₋₁₀ aryl group optionally having a halogen atom, a C₁₋₆ alkyl group, a hydroxy group, or a C₁₋₆ alkoxy group,
R₂ is an alkyl group having 1 - 6 carbon atoms (C₁₋₆ alkyl group),
R₃ is a hydroxy group, and
n is 0, 1, or 2.

### [Compound (1-5)]

A compound represented by the formula (I) wherein, in the aforementioned formula (I),
X is -NHCO-,
R₁ is -Y-NH-Z-Ar, wherein Y is an alkylene group having 1 - 4 carbon atoms (C₁₋₄ alkylene group) and substituted by an alkyl group having 1 - 4 carbon atoms (C₁₋₄ alkyl group), Z is a single bond, Ar is a phenyl group or naphthyl group, each optionally having a halogen atom, a C₁₋₄ alkyl group, a hydroxy group, or a C₁₋₄ alkoxy group,
R₂ is an alkyl group having 1 - 4 carbon atoms (C₁₋₄ alkyl group), and
n is 0.

### [Compound (1-6)]

A compound represented by the formula (I) wherein, in the aforementioned formula (I),
X is -NHCO-,
R₁ is -Y-NH-Z-Ar, wherein Y is a methylene group substituted by a methyl group or an ethyl group, Z is a single bond, Ar is a phenyl group optionally having a halogen atom, a methyl group, an ethyl group, a hydroxy group, a methoxy group, or an ethoxy group (preferably, a phenyl group optionally having a hydroxy group or a methyl group),
R₂ is a methyl group, an ethyl group, or an isobutyl group, and n is 0.

### [Compound (I-7)]

A compound represented by the formula (I) wherein, in the aforementioned formula (I),
X is -NHCO-,
R₁ is -Y-NH-Z-Ar, wherein Y is a methylene group substituted by a methyl group or an ethyl group, Z is a single bond, Ar is a phenyl group optionally having a halogen atom (preferably, a fluorine atom, a bromine atom) or a hydroxy group,
R₂ is a methyl group, an ethyl group, or an isobutyl group, and n is 0.

Specific examples of a particularly preferable compound represented by the formula (I) include the compounds described in the below-mentioned Table 1.

The subject of application of the therapeutic agent of the present invention is not particularly limited as long as it is an animal that may be affected with a corneal disease. However, the subject of application of the therapeutic agent of the present invention is generally a mammal including, for example, human, dog, cat, bovine, sheep, pig, horse, mouse, rat, and the like. Preferably, the subject of application of the therapeutic agent of the present invention is a human.

The therapeutic agent of the present invention can be in any dosage form at the time of provision or storage. The therapeutic agent of the present invention may have a dosage form of solid, liquid, gel, or the like. The therapeutic agent of the present invention can be generally administered as an oral administration agents such as tablet, capsule, powder, granule, pill, syrup or the like, eye drop, ophthalmic ointment, percutaneous absorbent, or ophthalmic injection. Among these, eye drop, ophthalmic ointment, and ophthalmic injection are preferable, and eye drop is most preferable because they can be easily administered locally to the eye with minimal invasiveness. The therapeutic agent of the present invention can be administered alone or as a mixture with other therapeutic drugs. They may be administered as a single compound represented by the formula (I), but are generally administered in the form of a pharmaceutical composition. These preparations can be produced by a conventional method by adding pharmacologically and pharmaceutically acceptable additives. That is, conventional additives such as excipients, lubricants, binders, disintegrants, wetting agents, plasticizers, coating agents and the like can be used for oral preparations. Oral liquid may be in the form of aqueous or oily suspension, solution, emulsion, syrup, elixir, or the like, or may be provided as dry syrup which is prepared using water or other suitable solvent prior to use. The aforementioned liquid can contain conventional additives such as suspending agent, flavor, diluent, or emulsifier. For ophthalmic injection, preparation components such as dissolving agent or solubilizing agent (e.g., distilled water for injection, saline, 5% glucose solution, propylene glycol, and the like), pH adjuster, isotonic agent, stabilizer, and the like are used so as to form an aqueous dosage form or a dosage form for dissolution before use.

As the ophthalmic ointment, a conventional base such as white petrolatum, liquid paraffin, and the like can be prepared. Eye drops can be prepared by using isotonic agents such as sodium chloride, potassium chloride, glycerol, propylene glycol and the like, buffering agents such as sodium phosphate, sodium acetate, sodium borate, sodium carbonate and the like, surfactants such as polyoxyethylene sorbitan fatty acid ester, polyoxyl stearate 40, polyoxyethylene polyoxypropylene glycol, polyoxyethylene castor oil, polyoxyethylene hydrogenated castor oil and the like, stabilizers such as disodium edetate, sodium citrate and the like, antiseptics such as benzalkonium chloride, sorbic acid, methyl parahydroxybenzoate and the like, thickening agents such as methylcellulose, hydroxymethylcellulose, polyvinylpyrrolidone and the like, antioxidants such as ascorbic acid, tocopherol and the like, and the like as necessary. The pH may be in the range acceptable for ophthalmological preparations, and is preferably in the range of 4 to 8. When adjusting the pH, hydrochloric acid, phosphoric acid, citric acid, sodium hydroxide, potassium hydroxide, sodium carbonate and the like can be used. The following shows Formulation Examples of the therapeutic agent of the present invention, but the present invention is not limited thereto.

### Formulation Example 1

An ophthalmic ointment (100 g) containing the following components is produced.

### Components

| | |
|---|---|
| Compound represented by the formula (I) | 300 mg |
| Liquid paraffin | 10 g |
| White petrolatum | q.s. |

### Formulation Example 2

An eye drop (100 mL) containing the following components is produced.

### Components

| | |
|---|---|
| Compound represented by the formula (I) | 500 mg |
| Sodium hydroxide | 900 mg |
| Sterile purified water | q.s. |

### Formulation Example 3

An eye drop (100 mL) containing the following components is produced.

### Components

| | |
|---|---|
| Compound represented by the formula (I) | 500 mg |
| Polyoxyl 35 castor oil | 90 mg |
| Hydrochloric acid | q.s. |
| Sodium hydroxide | q.s. |
| Sterile purified water | q.s. |

### Formulation Example 4

A tablet containing the following components is produced.

### Components

| | |
|---|---|
| Compound represented by the formula (I) | 10 mg |
| Lactose | 90 mg |
| Microcrystalline cellulose | 30 mg |
| Magnesium stearate | 5 mg |
| CMC-Na | 15 mg |
| total | 150 mg |

### Formulation Example 5

A preparation for injection containing the following components is produced.

| | |
|---|---|
| Compound represented by the formula (I) | 100 mg |
| Saturated fatty acid glyceride | 1000 mL |

The content of the specific compound or a salt thereof in the therapeutic agent of the present invention is not particularly limited. In the case of eye drop, the lower limit is preferably 0.001%(w/v), more preferably 0.01%(w/v), further preferably 0.1%(w/v), most preferably 0.5%(w/v). The upper limit is preferably 5%(w/v), more preferably 3%(w/v), further preferably 2%(w/v), most preferably 1%(w/v).

When the therapeutic agent of the present invention is administered to humans, the dose of the specific compound or a salt thereof is not particularly limited and can be appropriately determined according to the age, sex, and state of the patient. For example, in the case of topical ocular administration, the dose is 0.00001 - 100 mg, preferably 0.001 - 10 mg, more preferably 0.01 - 1 mg, most preferably 0.1 - 0.5 mg, per day. The administration can be a single dose administration or multiple dose administration. For example, when the therapeutic agent of the present invention is an eye drop, a single dose of 1 - 5 drops, more preferably 1 - 2 drops, most preferably 1 drop, can be administered 1 - 3 times a day, more preferably 1 - 2 times a day, most preferably once a day. Here, one drop is generally 0.01 - 0.1 mL. These numerical values are mere examples, and the dose is determined according to the patient's symptoms.

A derivative of the specific compound used in the invention that has a chemically or metabolically degradable group that produces the pharmacologically active compound of the present invention by solvolysis or under physiological conditions in vivo can be used as a prodrug. Methods for selecting and producing a suitable prodrug are described, for example, in Design of Prodrugs (Elsevier, Amsterdam 1985). In the present invention, when the compound has a hydroxy group, an acyloxy derivative obtained by reacting the compound with an appropriate acyl halide or an appropriate acid anhydride is exemplified as a prodrug. Acyloxy particularly preferred as the prodrug includes, for example, -OCOC₂H₅, -OCO(t-Bu), - OCOC₁₅H₃₁, -OCO(m-CO₂Na-Ph) , -OCOCH₂CH₂CO₂Na, -OCOCH(NH₂)CH₃, - OCOCH₂N(CH₃)₂ and the like. When the compound forming the present invention has an amino group, an amide derivative produced by reacting a compound having an amino group with an appropriate acid halide or an appropriate mixed acid anhydride is exemplified as the prodrug. Amide particularly preferred as the prodrug includes, for example, -NHCO (CH₂) ₂₀OCH₃, - NHCOCH(NH₂)CH₃ and the like.

Examples of the corneal diseases to which the therapeutic agent of the present invention is applied include corneal epithelium disorder, superficial punctate keratopathy, cornea erosion, corneal ulcer, dry eye, corneal epithelial abrasion, aniridia, pterygium, sclerocornea, cornea dystrophy, corneitis, limbal stem cell deficiency, chemical burn, burn, Stevens-Johnson syndrome, ocular pemphigoid, diabetes keratopathy, bullous keratopathy, corneal endothelitis, Fuchs' corneal endothelial dystrophy, cornea guttata, iridocorneal endothelial syndrome, post-corneal transplantation graft failure, and the like.

In the therapeutic agent of the present invention, the main action mechanism for the treatment of corneal diseases is promotion of the proliferation of corneal cells (corneal epithelial cells and/or corneal endothelial cells). Therefore, the therapeutic agent of the present invention can be rephrased as a proliferation promoter of corneal cells (corneal epithelial cells and/or corneal endothelial cells) (hereinafter to be also referred to as "the proliferation promoter of the present invention"). The proliferation promoter of the present invention may be applied to the subject of application of the therapeutic agent of the present invention, or may be added to a culture medium for test or transplantation of corneal cells.

### [2. Treatment method of corneal disease]

The present invention also provides a method for treating a corneal disease, including administering an effective amount of a compound represented by the formula (I) or a salt thereof to a subject in need thereof (hereinafter sometimes referred to as "the treatment method of the present invention").

In the treatment method of the present invention, the compound represented by the formula (I), treatment target, dose, administration route, administration frequency, applicable corneal diseases, etc. are the same as those described in [1. Therapeutic agent for corneal diseases].

### [3. Method for promoting proliferation of corneal cells]

The present invention also provides a method for promoting proliferation of corneal epithelial cells and corneal endothelial cells, including using a medium composition containing the aforementioned compound represented by the formula (I) or a salt thereof (hereinafter sometimes to be referred to as "the medium of the present invention", etc.). Using the method for promoting proliferation of corneal epithelial cells and corneal endothelial cells of the present invention, promotion of the proliferation of corneal epithelial cells and corneal endothelial cells and/or promotion of the formation of cell aggregates (spheres) can be achieved.

The concentration of the specific compound contained in the medium of the present invention as an active ingredient is not particularly limited as long as the desired effect of the present invention is obtained. For example, the lower limit value of the concentration may be generally not less than 0.001 µM, preferably not less than 0.01 µM, more preferably not less than 0.1 µM, further preferably not less than 1 µM, particularly preferably not less than 10 µM. , The upper limit value of the concentration may be generally not more than 100 µM, preferably not more than 50 µM, particularly preferably not more than 10 µM.

The medium of the present invention may have the same composition as that of a known medium except that the compound used in the present invention or the composition of the present invention is blended.

In one embodiment, the medium of the present invention can be prepared by adding the specific compound used in the present invention or a composition containing the specific compound (hereinafter to be also referred to as "the composition of the present invention") to a commercially available medium. Examples of the component of the composition include water, saline, dimethyl sulfoxide (DMSO), glycerol, propylene glycol, butyleneglycol, various alcohols such as methanol, ethanol, butanol, propanol and the like, and the like. In addition, the composition of the present invention may be subjected to a sterilization treatment as necessary. The sterilization method is not particularly limited and, for example, radiation sterilization, ethylene oxide gas sterilization, autoclave sterilization, filter sterilization and the like can be mentioned. The material of the filter portion when filter sterilization (hereinafter sometimes to be also referred to as "filtration sterilization") is not particularly limited and, for example, glass fiber, nylon, PES (polyether sulfone), hydrophilic PVDF (polyvinylidene fluoride), cellulose mixed ester, cellulose acetate, polytetrafluoroethylene and the like can be mentioned. The composition of the present invention may have any dosage form at the time of provision or storage. The composition may be a formulated solid such as tablet, pill, capsule, granule, a liquid such as a solution or suspension dissolved in an appropriate solvent or solubilizer, or a state bonded to a substrate or carrier.

A commercially available medium which can be used as the medium of the present invention by adding the specific compound or the composition of the present invention used in the present invention is not particularly limited as long as the desired effect is exerted. For example, media such as Dulbecco's modified Eagle's medium (DMEM), Ham F12 medium (Ham's Nutrient Mixture F12), DMEM/F12 medium, McCoy's 5A medium, Eagle MEM (Eagle's Minimum Essential medium; EMEM), αMEM (alpha Modified Eagle's Minimum Essential medium; αMEM), MEM (Minimum Essential medium), RPMI1640 medium, Iscove's Modified Dulbecco medium (IMDM), MCDB131 medium, Williams E medium, IPL41 medium, Fischer's medium, StemPro34 (manufactured by Invitrogen Corp.), X-VIVO 10 (manufactured by Cambrex Corporation), X-VIVO 15 (manufactured by Cambrex Corporation), HPGM (manufactured by Cambrex Corporation), StemlineII (manufactured by Sigma-Aldrich Co. LLC), QBSF-60 (manufactured by Quality Biological, Inc.), StemProhESCSFM (manufactured by Invitrogen Corp.), CSTI-7 medium (manufactured by Cell Science & Technology Institute, Inc.), MesenPRO RS medium (manufactured by Gibco), MF-medium (registered trade mark) mesenchymal stem cellular proliferation medium (manufactured by TOYOBO CO., LTD.), mesenchymal stem cell medium (manufactured by PromoCell GmbH.), Sf-900II (manufactured by Invitrogen Corp.), Opti-Pro (manufactured by Invitrogen Corp.), corneal epithelial cell basal medium (ATCC (registered trade mark), PCS-700-030 (registered trade mark)), Prigrow medium (manufactured by Applied Biological Materials), Eagle's minimal essential medium (Basal medium Eagle), BGJb medium, CMRL1066 medium and the like can be mentioned. In addition, a medium obtained by adding a polysaccharide such as deacylated gellan gum or the like to these media to form a three-dimensional cell culture medium can be used. Examples of such medium for three-dimensional cell culture include, but are not limited to, FCeM (registered trade mark) (manufactured by Nissan Chemical Corporation).

In addition, sodium, potassium, calcium, magnesium, phosphorus, chlorine, various amino acids, various vitamins, antibiotic, serum, fatty acid, sugar, cell growth factor, differentiation-inducing factor, cell adhesion factor, antibody, enzyme, cytokine, hormone, lectin, extracellular matrix, physiologically active substance and the like may be added to the above-mentioned media according to the purposes. Specific examples include, but are not limited to, basic fibroblast growth factor, transforming growth factor-β, epidermal growth factor, insulin-like growth factor, Wnt protein, interferons, interleukins, vascular endothelial cell growth factor, albumin, insulin, β-mercaptoethanol, Knockout Serum Replacement (KSR, manufactured by Thermo Fisher Scientific), Glutamax (manufactured by Thermo Fisher Scientific), hydrocortisone, epinephrine, L-glutamine, pyruvic acid, selenous acid and the like.

Cell culture in the medium of the present invention can be performed using culture containers generally used for cell culture, such as petri dish, flask, plastic bag, Teflon (registered trade mark) bag, dish, petri dish, dish for tissue culture, multidish, microplate, microwell plate, multiplate, multiwell plate, chamber slide, tube, tray, culture bag, roller bottle, and the like. These culture containers may be coated with an extracellular matrix such as collagen, gelatin, laminin, fibronectin, vitronectin, Matrigel (registered trade mark), poly-L-ornithine/laminin, poly-D-lysine, and the like. When forming cell aggregates (spheres), it is desirable that these culture containers are low cellular adhesive so that the cells will not adhere to the culture containers. As the low cellular adhesive culture container, one in which the surface of the culture container is not artificially treated (e.g., coating treatment with extracellular matrix, etc.) for the purpose of improving adhesiveness to cells, or one in which the surface of the culture container is artificially treated for the purpose of reducing adhesiveness to cells can be used. Examples of such container include, but are not limited to, SUMILON celltight plate (manufactured by SUMITOMO BAKELITE CO., LTD.), PrimeSurface (registered trade mark) plate (manufactured by SUMITOMO BAKELITE CO., LTD.), Super Low Contact Surface Plate (manufactured by Corning Incorporated), Nunclon Sphera plate (manufactured by Thermo Fisher Scientific) and the like.

Examples of the cell type in which cell proliferation promotion and the like are achieved by the medium of the present invention include corneal epithelial cells and corneal endothelial cells. For example, corneal endothelial cells can be obtained by detaching the Descemet's membrane from the corneal tissue and separating the endothelial cells by an enzymatic treatment. These cells may be genetically engineered by a virus, genome editing, or the like. In addition, corneal epithelial cells and corneal endothelial cells obtained by inducing differentiation from pluripotent stem cells such as embryonic stem cells, induced pluripotent stem cells, and the like can also be preferably used. While the origin of these cells is not particularly limited, the cells derived from mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee, human and the like are preferable.

As described above, since the medium of the present invention can promote proliferation of corneal cells (corneal epithelial cell and/or corneal endothelial cell), it can also be paraphrased as a medium composition for promoting the proliferation of corneal cells (corneal epithelial cells and/or corneal endothelial cells).

### [4. Production method of corneal cells]

The present invention also provides a method for producing corneal epithelial cells and corneal endothelial cells, including using a medium containing an effective amount of a compound represented by the aforementioned formula (I) or a salt thereof (hereinafter sometimes to be referred to as "the production method of the present invention"). The corneal epithelial cells and corneal endothelial cells obtained by the production method of the present invention can be preferably used as cells for transplantation in the treatment of corneal diseases.

In the production method of the present invention, the compound represented by the formula (I), the amount thereof to be added, the cells to be cultured, the corneal disease to be the target of the transplantation treatment using the obtained cells and the like are the same as those described in [1. Therapeutic agent for corneal diseases] and [3. Method for promoting proliferation of corneal cell].

The cell culture conditions (e.g., temperature, carbon dioxide concentration, culture period, etc.) in the production method of the present invention may be those of a method known per se, or may be appropriately modified according to the purposes. For example, the temperature when culturing the cells is generally 25°C - 39°C, preferably 33°C - 39°C (e.g., 37°C), for animal cells. The carbon dioxide concentration is generally 4% by volume - 10% by volume, preferably 4% by volume - 6% by volume, in the culture atmosphere. The culture period is generally 1 to 35 days, and can be appropriately set according to the purpose of culturing.

The present invention will be described in more detail in the following Examples, but the present invention is not limited to these examples.

### [Example]

The compound shown by the formula (I) can be produced by the method described in WO 2019/022222 or WO 2019/235569, or a method analogous thereto. Examples of the compounds used in the Examples of the present invention are shown in Table 1, but the compounds that can be used in the present invention are not limited to those.

In the Table, Me means methyl, and similarly in the following, Et means ethyl. (R) for R₄ means R-configuration, and (rac) means a racemate. R₅ is a substituent of the aryl group, and the substituent of the aryl group is the same as that mentioned above. In R₅, "-" shows unsubstituted. The number indicated in the structural formula shows the substitution position of R₅. n is 0, 1, or 2, and m is 0, 1, 2, 3, 4, or 5. When R₅ is present in plurality, each R₅ may be the same or different.

### [TABLE 1]

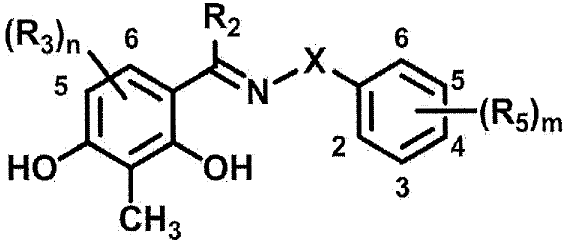

**[Table 1]**

| compound No. | R₂ | n | X | R₄ | R₅ | m |
|---|---|---|---|---|---|---|
| k-1:B-1 | Et | 0 | -NH-C(O)-CH(R₄)-NH- | Me(rac) | - | 0 |
| k-1:J-1 | Et | 0 | -NH-C(O)-NH-CH(R₄)- | H | 3-OH | 1 |
| k-1:D-1 | Et | 0 | -NH-C(O)-CH(R₄)-NH- | H | 3-OH | 1 |
| k-1:H-1 | Et | 0 | -NH-C(O)-CH(R₄)-NH- | H | - | 0 |
| k-1:I-10 | Et | 0 | -NH-C(O)-NH-CH(R₄) - | Me(rac) | 3-OH | 1 |
| GA-002A | Et | 0 | -NH-C(O)-CH(R₄)-NH- | Me(R) | - | 0 |
| GA-005A | Et | 0 | -NH-C(O)-CH(R₄)-NH- | Me(R) | 3-OH | 1 |
| CE-001 | Et | 0 | -NH-C(O)-NH-CH(R₄)- | H | 3-OH, 5-F | 2 |
| CE-002 | Et | 0 | -NH-C(O)-NH-CH(R₄)- | H | 3-OH, 4-F | 2 |
| CE-003 | Et | 0 | -NH-C(O)-NH-CH(R₄)- | H | 3-OH, 5-Br | 2 |

Among the compounds described in the above-mentioned Table 1, k-1:B-1, k-1:J-1, k-1:D-1, k-1:H-1, k-1:I-10, GA-002A and GA-005A are described in WO 2019/022222 or WO 2019/235569, and CE-001, CE-002 and CE-003 are novel compounds.

The synthetic method and the structural formulas of the novel compounds of the present invention are shown below. ¹H-NMR shows proton nuclear magnetic resonance spectrum, and measured at 270 MHz in deuterated dimethyl sulfoxide. The chemical shift value is indicated with the value of tetramethylsilane as 0.00 ppm. "s" means singlet, and similarly in the following, "brs" means broad singlet, "d" means doublet, "dd" means double doublet, "t" means triplet, "q" means quartet, and "m" means multiplet.

### [Synthetic Example 1] Synthesis of CE-001

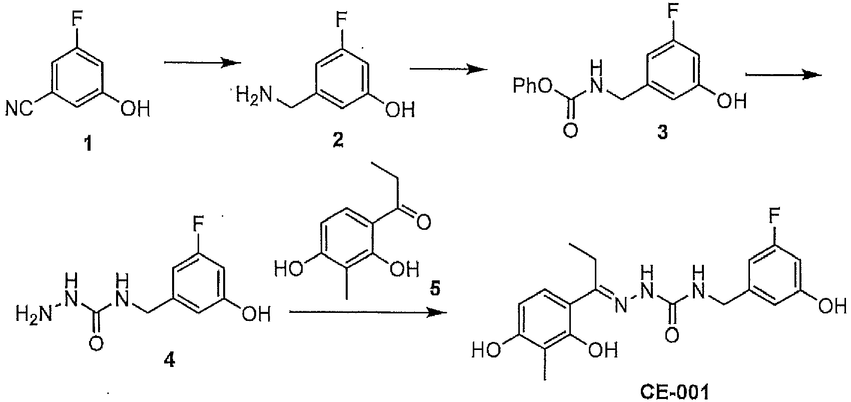

Lithium aluminum hydride (554 mg, 14.6 mmol) was suspended in THF (20 mL) under ice-cooling, 3-fluoro-5-hydroxybenzonitrile (1) (1.0 g, 7.3 mmol) was added, and the mixture was stirred at room temperature for 2 hr and at 60°C for 18 hr. The mixture was allowed to cool, lithium aluminum hydride (830 mg, 21.9 mmol) was added, and the mixture was further stirred for 4 hr. Under ice-cooling, distilled water (1.4 mL), 15% aqueous sodium hydroxide solution (1.4 mL), and distilled water (4.2 mL) were successively added, and the precipitated solid was filtered off by celite filtration. The filtrate was concentrated under reduced pressure to give compound (2) (726 mg, 5.14 mmol, yield 70%) as a colorless solid. The obtained compound (2) (726 mg, 5.14 mmol) was dissolved in methylene chloride (6.5 mL) and distilled water (6.5 mL), and phenyl chloroformate (1.3 mL, 10 mmol) was slowly added dropwise under ice-cooling. The mixture was stirred at room temperature for 3 hr and partitioned. The organic layer was dried over anhydrous magnesium sulfate, filtered, and concentrated under reduced pressure. The obtained residue was purified by medium-pressure silica gel column chromatography (silica gel 60 g, ethyl acetate/hexane=0/100 - 50/50) to give compound (3) (442 mg, 1.69 mmol, yield 33%) as a colorless solid. The obtained compound (3) (0.44 g, 1.7 mmol) was dissolved in ethanol (3.4 mL), hydrazine monohydrate (0.41 mL, 8.5 mmol) was added, and the mixture was stirred at 60°C for 18 hr. The reaction solution was concentrated under reduced pressure, and azeotropically distilled with toluene to give compound (4) (273 mg, 1.37 mmol, yield 81%). The obtained compound (4) (144 mg, 0.723 mmol) and 2',4'-dihydroxy-3'-methylpropiophenone (5) (100 mg, 0.55 mmol) were dissolved in DMSO (1.1 mL), and the mixture was stirred at 100°C for 3 days. The mixture was allowed to cool, and the reaction solution was directly purified by medium-pressure silica gel column chromatography (silica gel 30 g, ethyl acetate/hexane=50/50 - 100/0), and the obtained solid was washed by suspending in water to give CE-001 (99.6 mg, 0.276 mmol, yield 50%) as a colorless solid. ¹H-NMR(270MHz); δ 13.41(s, 1H), 9.87(s, 1H), 9.61(s, 1H), 9.54(s, 1H), 7.17(d, J=8.1Hz, 1H), 6.84(t, J=8.1Hz, 1H), 6.60-6.40(m, 3H), 6.37(d, J=8.1Hz, 1H), 4.26(d, J=8.1Hz, 2H), 2.66(q, J=8.1Hz, 2H), 1.97(s, 3H), 1.08(t, J=8.1Hz, 3H).

### [Synthetic Example 2] Synthesis of CE-002

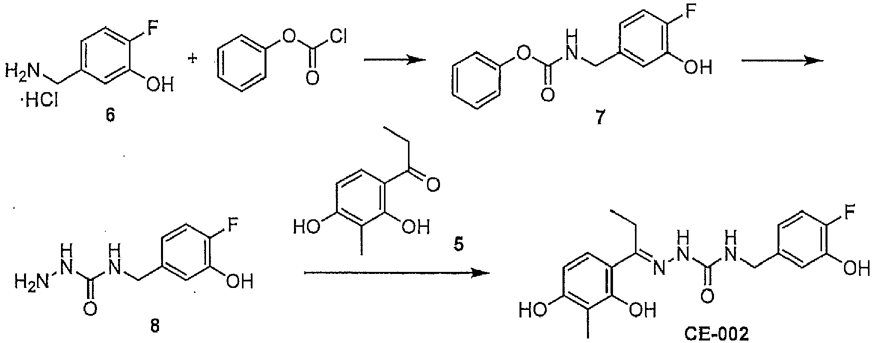

5-Aminomethyl-2-fluorophenol hydrochloride (6) (0.50 g, 2.1 mmol) was suspended in methylene chloride (5.0 mL) and water (5.0 mL), and sodium hydrogen carbonate (1.16 g, 14.0 mmol) was added. Under ice-cooling, phenyl chloroformate (0.58 mL, 5.0 mmol) was slowly added dropwise, and the mixture was stirred at room temperature for 2.5 hr. Ethyl acetate (30 mL) was added for partitioning, and the mixture was extracted with ethyl acetate (20 mLx2), dried over anhydrous sodium sulfate, filtered, and concentrated under reduced pressure to give compound (7) (0.95 g) as a colorless solid. The obtained compound (7) (0.95 g) was suspended in ethanol (4.0 mL), hydrazine monohydrate (1.0 mL, 21 mmol) was added, and the mixture was stirred at 60°C for 5 hr. The reaction solution was concentrated under reduced pressure, and the obtained pale-yellow solid was washed with methylene chloride, and purified by medium-pressure silica gel column chromatography (silica gel 30 g, methanol/methylene chloride =1/99 - 10/90) to give compound (8) (0.41 g, 2.0 mmol, 2 step yield 98%) as a pale-yellow solid. The obtained compound (8) (144 mg, 0.72 mmol) and 2',4'-dihydroxy-3'-methylpropiophenone (5) (100 mg, 0.55 mmol) were dissolved in DMSO (1.0 mL), and the mixture was stirred at 100°C for 1 day. The mixture was allowed to cool, and the reaction solution was directly purified by medium-pressure silica gel column chromatography (silica gel 30 g, methanol/methylene chloride =1/99 - 5/95). To the obtained purified product was added water, and the precipitated solid was collected by filtration, and washed by suspending in methylene chloride to give CE-002 (96.4 mg, 0.267 mmol, yield 48%) as a colorless solid. ¹H-NMR(270MHz); δ 13.43(s, 1H), 9.82(brs, 1H), 9.59(s, 1H), 9.55(brs, 1H), 7.17(d, J=8.1Hz, 1H), 7.11-7.00(m, 1H), 6.91(d, J=10.8Hz, 1H), 6.79(t, J=8.1Hz, 1H), 6.75-6.70(m, 1H), 6.37(d, J=8.1Hz, 1H), 4.23(d, J=5.4Hz, 2H), 2.65(q, J=8.1Hz, 2H), 1.97(s, 3H), 1.07(t, J=8.1Hz, 3H).

### [Synthetic Example 3] Synthesis of CE-003

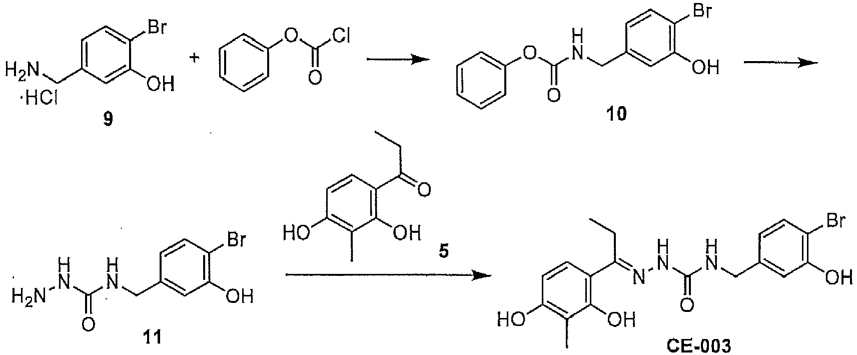

5-Aminomethyl-2-bromophenol hydrochloride (9) (0.48 g, 2.0 mmol) was suspended in methylene chloride (5.0 mL) and water (5.0 mL), and sodium hydrogen carbonate (1.16 g, 14.0 mmol) was added. Under ice-cooling, phenyl chloroformate (0.56 mL, 4.0 mmol) was slowly added dropwise, and the mixture was stirred at room temperature for one day. Water (5.0 mL) and ethyl acetate (30 mL) were added for partitioning, and the mixture was extracted with ethyl acetate (5 mLx3), dried over anhydrous sodium sulfate, filtered, and concentrated under reduced pressure to give compound (10) (1.0 g) as a yellow liquid. The obtained compound (10) (1.0 g) was suspended in ethanol (4.0 mL), hydrazine monohydrate (1.0 mL, 21 mmol) was added, and the mixture was stirred at 60°C for 5 hr. The reaction solution was concentrated under reduced pressure, and the obtained pale-yellow solid was washed with methylene chloride, and purified by medium-pressure silica gel column chromatography (silica gel 30 g, methanol/methylene chloride =1/99 - 10/90) to give compound (11) (0.42g, 1.6 mmol, 2 step yield 80%) as a pale-yellow solid. The obtained compound (11) (188 mg, 0.72 mmol) and 2',4'-dihydroxy-3'-methylpropiophenone (5) (100 mg, 0.55 mmol) were dissolved in DMSO (1.1 mL), and the mixture was stirred at 100°C for 4 days. The mixture was allowed to cool, and the reaction solution was directly purified by medium-pressure silica gel column chromatography (silica gel 30 g, methanol/methylene chloride =1/99 - 5/95). To the obtained purified product was added water, and the precipitated solid was collected by filtration, and washed by suspending in methylene chloride to give CE-003 (159 mg, 0.377 mmol, yield 68%) as a colorless solid. ¹H-NMR(270MHz); δ 13.42(s, 1H), 10.20(brs, 1H), 9.62(s, 1H), 9.55(brs, 1H), 7.42(d, J=8.1Hz, 1H), 7.17(d, J=8.1Hz, 1H), 6.91(s, 1H), 6.83(t, J=8.1Hz, 1H), 6.79(d, J=8.1Hz, 1H), 6.37(d, J=8.1Hz, 1H), 4.24(d, J=5.4Hz, 2H), 2.65(q, J=8.1Hz, 2H), 1.97(s, 3H), 1.08(t, J=8.1Hz, 3H).

### [Example 1] Cell proliferation test using human corneal epithelial cells

As human primary corneal epithelial cells, {American Type Culture Collection (ATCC), #PCS-700-010} was precultured by a single layer culture method using a corneal epithelial cell basal medium (ATCC, #PCS-700-030) added with Corneal Epithelial Cell Growth Kit (ATCC, #PCS-700-040). After suspending the corneal epithelial cells in the same medium, the specific compounds used in the present invention and dissolved in DMSO were respectively added to a final concentration of 10 µM, and seeded in a 96well flat bottom plate (Corning Incorporated, #3585) and an imaging analysis plate (Perkin Elmer Inc., CellCarrier-96Ultra) at 700 cells/64 µL/well to 1600 cells/64 µL/well. The cells were cultured in a 5% CO₂ incubator at 37°C for 4 days. As a control, DMSO was added to a final concentration of 0.1%. After culturing, the number of viable cells was measured using ATP reagent (Promega KK, CellTiter-Glo (registered trade mark) Luminescent Cell Viability Assay). The ATP reagent was added to the culture medium in the flat bottom plate and suspended at 64 µL/well. The suspension (100 pL/well) was transferred to a white plate for assay (Corning Incorporated, #3912). After allowing to stand at room temperature for 10 min, the amount of luminescence was measured using a plate reader (Perkin Elmer, EnSpire). The cells cultured on the imaging analysis plate were fixed by exchanging the medium with a 4% paraformaldehyde solution and allowing to stand at room temperature for 10 min. The fixed cells were subjected to fluorescence immunostaining using p63-α antibody (Cell Signaling Technology, #4892) and AlexaFluor647 modified anti-rabbit IgG antibody (Thermo Fisher Scientific, #A-21244) and according to the recommended protocol of Cell Signaling Technology, Inc. Furthermore, cell nucleus was stained with 10 pg/mL Hoechst33342 solution. The stained cells were imaged in 9 fields per well using a 20x objective lens of OperattaCLS.

The relative value of the measured amount of luminescence with respect to the control group (DMSO) was calculated to find that the amount of luminescence increased by 1.5 times or more by the addition of the following compound. That is, the number of cells increased by 1.5 times or more by the addition of the following compounds. In addition, as a result of imaging analysis, all the cells were p63α-positive when k-1:B-1 or k-1:J-1 was added. Therefrom it was shown that the composition of the present invention has a proliferation promoting effect on p63α-positive human corneal epithelial cells.

Compounds with cell number increased by 1.5 times or more:
k-1:B-1, k-1:J-1, k-1:D-1, GA-002A, GA-005A, CE-001, k-1:I-10, CE-002, CE-003

Compounds with cell number increased by 2 times or more: k-1:B-1, k-1:J-1, k-1:D-1, GA-005A, CE-001, k-1:I-10, CE-002

### [Example 2] Cell proliferation test using bovine corneal endothelial cells

Bovine corneal endothelial cell line BCE C/D-1b (American Type Culture Collection (ATCC), #CRL-2048) was precultured using DMEM (ATCC, #30-2002) added with 10% bovine serum (ATCC,#30-2030). Corneal endothelial cells were suspended in the same medium, and seeded in a 96 well flat bottom plate (Corning Incorporated, #3585) at 700 cells/90 µL/well in a single layer culture method (2D). In the three-dimensional culture method (3D), the cells were seeded in a 96 well U-bottom low adhesion plate (Corning Incorporated,#4520) at 700 cells/90 µL/well. Successively, the specific compounds used in the present invention and dissolved in DMSO were respectively added at 10 µL/well so as to achieve a final concentration of 5 µM or 10 µM, and the cells were cultured in a 5% CO₂ incubator at 37°C for 4 days. As a control, DMSO was added to a final concentration of 0.1%. After culturing, the number of viable cells was measured using ATP reagent (Promega KK, CellTiter-Glo (registered trade mark) Luminescent Cell Viability Assay). The ATP reagent was added to the culture medium at 100 µL/well. The suspension (100 µL/well) was transferred to a white plate for assay (Corning Incorporated, #3912). After allowing to stand at room temperature for 10 min, the amount of luminescence was measured using a plate reader (Perkin Elmer, EnSpire) .

The relative value of the measured amount of luminescence with respect to the control group (DMSO) in each of 2D and 3D was calculated to find that the amount of luminescence increased by 1.4 times or more in 2D and 2 times or more in 3D by the addition of the following compound. That is, the number of cells increased by 1.4 times or more by the addition of the following compounds. Therefrom it was shown that the composition of the present invention has a proliferation promoting effect on corneal endothelial cells.

Compounds with cell number increased by 1.4 times or more (2D) :
5 µM; GA-005A, CE-001
10 µM; k-1:B-1, k-1:J-1, k-1:H-1, k-1:I-10

Compounds with cell number increased by 2 times or more (3D) :
10 µM; k-1:B-1, k-1:J-1, k-1:D-1, GA-002A, GA-005A, CE-001, k-1:I-10, k-1:H-1

### [Industrial Applicability]

The diseases caused by a decrease in corneal epithelial cells and corneal endothelial cells can be treated by the administration of the therapeutic agent for corneal diseases of the present invention. Therefore, the therapeutic agent for corneal diseases of the present invention is preferably used for treating various corneal diseases and is extremely beneficial in the medical field.

This application is based on a patent application No. 2019-044854 filed in Japan (filing date: March 12, 2019), the contents of which are incorporated in full herein.

## Claims

1. A therapeutic agent for a corneal disease, comprising a compound represented by the following formula (I) or a salt thereof: 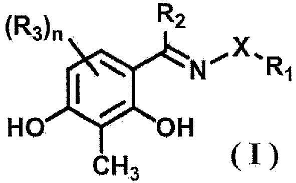 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.

2. The therapeutic agent according to claim 1, wherein
R₂ is an alkyl group having 1 - 4 carbon atoms,
n is 0,
Ar is a phenyl group or naphthyl group, each optionally having a hydroxy group or a C₁₋₄ alkyl group,
Y is a single bond, and
Z is an alkylene group having 1 - 4 carbon atoms.

3. The therapeutic agent according to claim 1, wherein
R₂ is an alkyl group having 1 - 4 carbon atoms,
n is 0,
Ar is a phenyl group or naphthyl group, each optionally having a halogen atom, a C₁₋₄ alkyl group, a hydroxy group, or a C₁₋₄ alkoxy group,
Y is an alkylene group having 1 - 4 carbon atoms and substituted by an alkyl group having 1 - 4 carbon atoms, and
Z is a single bond.

4. The therapeutic agent according to claim 1, wherein
R₂ is a methyl group, an ethyl group, or an isobutyl group,
n is 0,
Ar is a phenyl group optionally having a hydroxy group or a methyl group, and
Y is a methylene group optionally having a methyl group or an ethyl group.

5. The therapeutic agent according to claim 1, wherein
R₂ is a methyl group, an ethyl group, or an isobutyl group,
n is 0,
Ar is a phenyl group optionally having a hydroxy group or a halogen atom, and
Y is a methylene group optionally having a methyl group or an ethyl group.

6. The therapeutic agent according to claim 1, wherein
R₂ is a methyl group, an ethyl group, or an isobutyl group,
n is 0,
Ar is a phenyl group optionally having a hydroxy group or a methyl group, and
Y is a single bond.

7. The therapeutic agent according to claim 4, wherein Z is a single bond.

8. The therapeutic agent according to claim 5 or 6, wherein Z is a methylene group optionally having a methyl group or an ethyl group.

9. The therapeutic agent according to any one of claims 1 to 8, wherein the corneal disease is selected from the group consisting of corneal epithelium disorder, superficial punctate keratopathy, cornea erosion, corneal ulcer, dry eye, corneal epithelial abrasion, aniridia, pterygium, sclerocornea, cornea dystrophy, corneitis, limbal stem cell deficiency, chemical burn, burn, Stevens-Johnson syndrome, ocular pemphigoid, diabetes keratopathy, bullous keratopathy, corneal endothelitis, Fuchs' corneal endothelial dystrophy, cornea guttata, iridocorneal endothelial syndrome, and post-corneal transplantation graft failure.

10. A method for treating a corneal disease, comprising administering a compound represented by the following formula (I) or a salt thereof to a subject in need thereof: 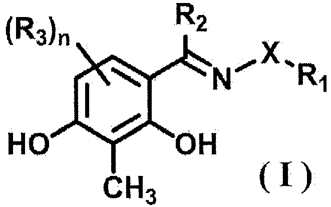 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.

11. A method for promoting the proliferation of a corneal epithelial cell or a corneal endothelial cell, comprising culturing the corneal epithelial cell or the corneal endothelial cell in a medium composition comprising a compound represented by the following formula (I) or a salt thereof: 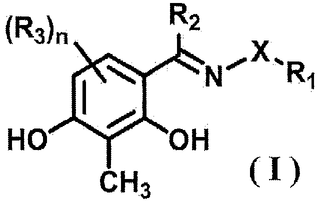 {wherein, X is -NHCO-, R₁ is -Y-NH-Z-Ar (wherein Y and Z are each independently a single bond, or an alkylene group having 1 - 6 carbon atoms and optionally having substituent(s), and Ar is an aryl group optionally having substituent(s)), R₂ is an alkyl group having 1 - 6 carbon atoms and optionally having substituent(s), R₃ is a hydroxy group, and n is 0, 1, or 2}.

12. A method for producing corneal epithelial cells or a corneal endothelial cells, comprising using the method according to claim 11.

13. A compound represented by the following formula: 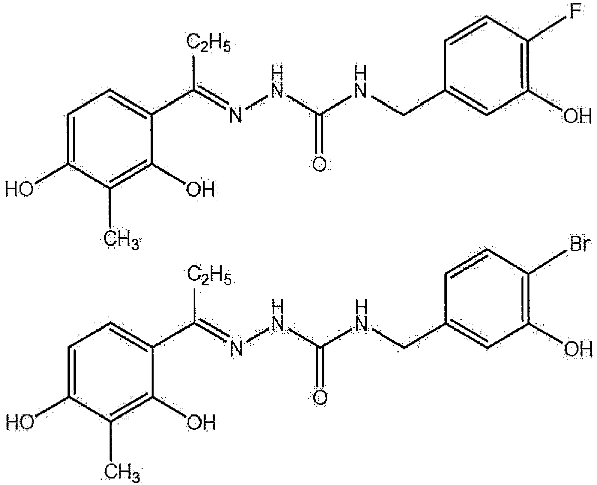 or or a salt thereof.
